(19) Europäisches Patentamt
European Patent Office
Office européen des brevets

(11) Publication number: **0 053 029**
**A1**

(12) # EUROPEAN PATENT APPLICATION

(21) Application number: 81305519.1

(22) Date of filing: 23.11.81

(51) Int. Cl.³: C 07 C 103/52
A 61 K 37/02

(30) Priority: 24.11.80 US 209337

(43) Date of publication of application:
02.06.82 Bulletin 82/22

(84) Designated Contracting States:
BE CH DE FR GB IT LI LU NL SE

(71) Applicant: PENINSULA LABORATORIES INCORPORATED
611 Taylor Way
Belmont California(US)

(72) Inventor: Chang, Jaw-Kang
1233 Lane Street
Belmont California 94002(US)

(74) Representative: Goldin, Douglas Michael et al,
J.A. KEMP & CO. 14, South Square Gray's Inn
London WC1R 5EU(GB)

(54) **Non enkephalin-like peptides, their production, compositions containing them, and their medical and veterinary use.**

(57) Non enkephalin-like tetra- and penta-peptides, especially Tyr-Pro-N-Me-Phe-D-Pro-$NH_2$, are useful as analgesics. They are prepared by conventional solid or liquid phase methods.

EP 0 053 029 A1

DESCRIPTION

TITLE: "NON ENKEPHALIN-LIKE PEPTIDES, THEIR PRODUCTION, COMPOSITIONS CONTAINING THEM, AND THEIR MEDICAL AND VETERINARY USE"

THIS INVENTION relates to new peptides exhibiting analgesic activity, to their preparation, to compositions containing them and to their medicinal and veterinary use.

Recently, morphine receptor and enkephalin receptor sites have been identified, see for example Kwen-Jen Chang and Pedro Cuatrecasas, The Journal of Biological Chemistry, vol. 254, No. 8, April 25, 1979, pp 2610-2618.

It has been speculated that a good analgesic activity would be exhibited if one could provide compounds which would more strongly bind to the morphine (μ) receptor site than to the enkephalin (δ) receptor site.

We have now discovered new peptides that bind substantially more strongly to the morphine (μ) receptor site than to the enkephalin (δ) site and also exhibit analgesic activity. Because of the strong binding to the μ sites, less compound in comparison with enkephalin analogue compounds is needed to achieve analgesic activity.

In addition, because of high selectivity to the morphine receptor site the new peptides can be expected to produce less side effects than morphine.

The present invention provides a peptide of the general formula (I):

$$A\text{-}B\text{-}C\text{-}Z\text{-}(R^2)_n \quad (I)$$

where n is 0 or 1.

A is Tyr, N-alkyl-Tyr where the alkyl group

has 1 to 4 carbon atoms, and is preferably methyl or ethyl,or N-alkenyl-Tyr where the alkenyl group has 2 to 4 carbon atoms, and is preferably allyl, the Tyr in each case having L chirality.

B is Pro(L), 3,4-dehydro-Pro(L), 4-hydroxy-Pro(L), Thz (L) or Pip (L).

C is Phe(L or D), N-alkyl-Phe (L or D), α-alkyl-Phe (L or D), in each of the above the alkyl group having 1 to 4 carbons and is preferably methyl.

Z is Pro (L or D), Leu (L or D), Thz (L or D), Tyr (L or D), Pip (L or D), Hse (L or D), Nva (L or D), 4-hydroxy-Pro (L or D), Met (L or D), Met($O_2$) (L or D), Ile (L or D), Nle (L or D), Met(O) (L or D), Gly, Abu (L or D), Val (L or D), Aib, 3,4-dehydro-Pro (L or D), cyclo-Leu (L or D)

$R^2$ is Gly-$NH_2$, $-OR^5$, (where $R^5$ is an alkyl group of 1 to 4 carbon atoms, preferably methyl or ethyl), or a group $-NR^6R^7$ (where $R^6$ and $R^7$ are the same or different and each is hydrogen or an alkyl group of 1 to 4 carbon atoms preferably methyl), and, when n is 0, the alcohols resulting from reduction of the peptide A-B-C-Z, and pharmaceutically acceptable salts thereof,

except that where n is 1 and A is Tyr and B is Pro and C is Phe, and Z is Pro, $R^2$ is not $NH_2$ and except that where n is 0, A is Tyr and B is Pro, and C is Phe, Z is not Pro.

In the above L and D define the chirality of the amino acid.

In the peptide formula representations used herein for peptides within the definition of formula (I) the amino-terminal amino acid is at the left side of the molecule and is represented by Tyr when the amino group is unsubstituted as is conventional in the art. When the

amino group of the tyrosine moiety is substituted, the substitution is indicated in the conventional manner, e.g. N-Me-Tyr- for N-methyltyrosine. Also as is conventional in the art, the carboxy-terminal amino acid is at the right side of the molecule and is represented by the appropriate amino acid symbol when the carboxy group is unsubstituted, i.e. -COOH, or by the appropriate amino acid symbol followed by the appropriate modifying symbols indicating the nature of the substitution for modified carboxy groups, e.g. $-NH_2$ indicates $-\overset{O}{\overset{\|}{C}}-NH_2$, -OR is $-\overset{O}{\overset{\|}{C}}-OR$ and -ol is $-CH_2OH$.

In this Specification, the following abbreviations, most of which are well-known and are commonly used in peptide chemistry are employed:

Abu-α-aminobutyric acid
Aib-α-amino-iso-butyric acid
Ala - alanine
Gly - glycine
Hse - homoserine
Ile - isoleucine
Leu - leucine
Met - methionine
Nle - norleucine
Nva - norvaline
Phe - phenylalanine
Tyr - tyrosine
Val - valine

Thz is 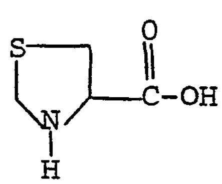

Pip is 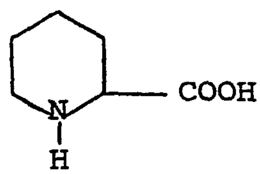 Pro-ol is 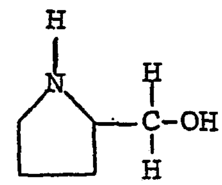

Reference may also be made to Biochemistry, 11, 1726 (1972) for the definition of the above abbreviations.

The most preferred compound for its analgesic activity is Tyr-Pro-N-Me-Phe-D-Pro-$NH_2$.

Other compounds of the invention having high analgesic activity are

Tyr-Pro-N-Me-Phe-D-Pro-ol

Tyr-Thz-N-Me-Phe-D-Pro-$NH_2$

Tyr-Pro-Phe-Thz-$NH_2$

Pharmaceutically acceptable salts of the invention include non-toxic acid addition salts where n is 1 and include the organic and inorganic acid addition salts, for example, those prepared from acids such as hydrochloric, sulphuric, sulphonic, tartaric, fumaric, hydrobromic, glycolic, citric, lactic, maleic, phosphoric, succinic, acetic, nitric, benzoic, ascorbic, p-toluenesulphonic, benzenesulphonic, naphthalenesulphonic, methanesulphonic, propionic and the like. Preferably, the acid addition salts are those prepared from hydrochloric acid, acetic acid or succinic acid. The above salts are prepared by conventional methods. Where n is 0, pharmaceutically acceptable salts include alkali metal salts, e.g. Na or K, or ammonium salts.

In the above and in this disclosure all references are to the L-amino acids and their radicals except in the case of glycine and Aib and unless otherwise indicated. The term "alkyl group of 1 to 4 carbon atoms" is intended to include methyl, ethyl, n-propyl, isopropyl, n-butyl, isobutyl, sec.-butyl and tert-butyl.

Examples of other peptides of the invention are:

Tyr-Pro-Phe-D-Leu-$NH_2$

Tyr-3,4-dehydro-Pro-N-Me-Phe-D-Pro-$NH_2$

Tyr-4-hydroxy-Pro-N-Me-Phe-D-Pro-$NH_2$

Tyr-Pip-N-Me-Phe-D-Pro-$NH_2$

Tyr-Pro-N-Me-Phe-D-Pro

N-Me-Tyr-Pro-N-Me-Phe-D-Pro-$NH_2$

Tyr-Pro-N-Me-Phe-Pro-$NH_2$

Tyr-Pro-N-Me-Phe-D-Pro-Gly-$NH_2$

In the acid addition salts of the peptides of this invention, the activity resides in the base and the particular salt is of less importance although for therapeutic purposes it is preferably pharmaceutically acceptable to the recipient. Likewise in the salts of the peptides (comprising the peptide as the carboxylated anion together with a cation) the identity of the cation is of less importance for therapeutic purposes.

The peptides of formula (I) and their salts and acid addition salts may be prepared by any of the methods known in the art for the preparation of peptide compounds. Thus they may be formed by the sequential coupling of appropriate amino acids using either classical methods of peptide synthesis or solid phase procedures, e.g. using a Schwarz/Mann Automatic Synthesizer, or by the initial preparation and subsequent coupling of peptide subunits.

Such reaction may be affected by, for example, activating the carboxylic acid group of the ingoing amino acid and protecting the non-reacting amino and carboxylic acid groups. Such techniques are standard in the peptide art. Details of suitable activating and protecting (masking) groups and of suitable reaction conditions (both for the coupling reaction and for the removal of

of protecting groups) giving the minimum of racemisation may be found in the following literature, all of which is incorporated herein by reference hereto, which is given purely by way of exemplification and which is intended to be neither exhaustive nor limiting.

(a) Published United Kingdom Patent Specifications Nos. 1,042,487; 1,048,086; and 1,281,383.

(b) Schroder and Luebke, "The Peptides" (Academic Press 1965).

(c) Bellean and Malek, J. Am. Chem. Soc., 90, 165 (1968)

(d) Tilak, Tetrahedron Letters, 849 (1970)

(e) Beyerman, Hlv. Chim. Acta., 56, 1729 (1973)

(f) Stewart and Young, "Solid Phase Peptide Synthesis" (W.H. Freeman and Co.) (1969).

Depending upon the reaction conditions the peptides of formula (I) are obtained in the form of the free base or as an acid addition salt or salt thereof. The acid addition salts may be converted into the free bases or salts of other acids, and the bases may be converted into acid addition salts thereof, by techniques well-known in the art. Likewise the peptides may be converted to salts thereof, and the salts converted to the peptides or to other salts, by well established techniques.

The peptides of formula (I), and their salts and acid addition salts may thus be prepared by condensing a reagent (III)

E-OH (III)

wherein E is A- or A-B or A-B-C- or, when n is 1 and $R^2$ is $GlyNH_2$, E may be A-B-C-Z- with a reagent (IV)

H - Y (IV)

wherein Y is $-B-C-Z-(R^2)_n$ or $-C-Z-(R^2)_n$ or

$-Z-(R^2)_n$ or $GlyNH_2$ respectively, the reagents (III) and (IV) being optionally protected and/or activated where and as appropriate; followed if necessary and as appropriate by one or both of the steps of deprotection of the product and conversion of the product into the base or a salt or an acid addition salt thereof.

It will also be appreciated that other *in situ* conversions of the peptides of formula (I) are possible. Thus the peptides wherein $R^2$ is a group $-NR^6R^7$ may be prepared by for example reaction of a peptide alkyl ester (wherein $R^2$ is $-OR^5$ where $R^5$ is alkyl) such as the methyl ester with ammonia or a mono- or diamine, as appropriate. The peptide esters may be prepared from the peptide acids ($R^2$ is $-OR^5$ where $R^5$ is hydrogen) by standard esterification procedures and the esters may be converted to the peptide acids by saponification. All these are conventional techniques in the peptide art and reference may be made to the literature referred to hereinabove for details of reaction conditions and of appropriate protection/ deprotection procedures.

Because of their analgesic activity and specificity in attachment to the morphine receptor already mentioned, the peptides of formula (I) together with their pharmaceutically acceptable salts and acid addition salts may be used in the treatment of mammals in the fields of both human and veterinary medicine in any condition where an analgesic is indicated. Specific indications that may be mentioned, by way of example, include the following:

The relief of pain (analgesia), for example pain arising from spasm of smooth muscle as in renal or biliary colic, pain due to terminal illness such as cancer, pain in the post-operative period, and obstetrical pain.

The amount of the peptides of formula (I) and their salts and acid addition salts, that is to say, for use as an analgesic (hereafter referred to as the active ingredient) will vary with the route of administration and with the nature and required extent of the desired effect, and will ultimately be at the discretion of the physician or veterinarian. In general, however, for the use as an analgesic the preferred dosage will be in the range 1 to 40 mg per kilogram bodyweight of mammal, e.g. dog, cat, humans, more preferably 1 to 10.0 mg/kg, most preferably 1.0 to 4.0 mg/kg, given two to three times daily (all dosages calculated with reference to the peptide base). For use as an analgesic for human a 3 mg. single dose given two or three times daily is preferred.

The active ingredients may be administered by any route appropriate to the effect to be achieved, suitable routes including oral, rectal, nasal, topical (buccal), vaginal and parenteral (including subcutaneous, intramuscular, intravenous and intracerebral, i.e. in the brain). It will be appreciated that the preferred route will vary with the effect to be achieved and thus for example in the relief of obstetrical pain administration directly into the spinal cord may be advantageous.

While it is possible for the active ingredients to be administered as the pure chemical, it is preferable to present them as a pharmaceutical formulation.

The formulations, both veterinary and for human use, of the present invention comprise an active ingredient, as above defined, together with one or more acceptable carriers therefor and optionally other therapeutic ingredients. The carrier(s) must be "pharmaceutically acceptable" in the sense of being compatible with the other ingredients of the formulation and not deleterious

to the recipient (patient) thereof. Desirably, the formulations should not include oxidising agents and other substances with which peptides are known to be incompatible.

The formulations include those suitable for oral, rectal, nasal, topical (buccal), vaginal or parenteral (including subcutaneous, intramuscular and intravenous) administration, although the most suitable route in any given case will depend upon for example the active ingredient and the condition to be treated. The formulations may conveniently be presented in unit dosage form and may be prepared by any of the methods well-known in the art of pharmacy. All methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product into the desired formulation.

Formulations of the present invention suitable for oral administration may be presented as discrete units such as capsules, cachets or tablets each containing a predetermined amount of the active ingredient; as a powder or granules; or as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine, the active ingredient in a free-flowing form such as a powder or granules, optionally mixed with a binder, lubricant, inert diluent, lubricating, surface

active or dispersing agent. Moulded tablets may be made by moulding in a suitable machine, a mixture of the powdered compound moistened with an inert liquid diluent.

Formulations for rectal administration may be presented as a suppository with the usual carriers such as cocoa butter, while a suitable formulation for nasal administration is nasal drops comprising the active ingredient in aqueous or oily solution.

Formulations suitable for topical administration in the mouth include lozenges comprising the active ingredient in a flavoured basis, usually sucrose and acacia or tragacanth; and pastilles comprising the active ingredient in an inert basis such as gelatin and glycerin, or sucrose and acacia.

Formulations suitable for vaginal administration may be presented as pessaries, creams, pastes or spray formulations containing in addition to the active ingredient such carriers as are known in the art to be appropriate.

Formulations suitable for parenteral administration conveniently comprise sterile aqueous solutions of the active ingredient, which solutions are preferably isotonic with the blood of the recipient. Such formulations may be conveniently prepared by dissolving solid active ingredient in water to produce an aqueous solution, and rendering said solution sterile and isotonic with the blood of the recipient. The formulations may be presented in unit- or in multi-dose containers, for example sealed ampoules or vials.

Formulations suitable for nasal administration wherein the carrier is a solid include a coarse powder having a particle size for example in the range 20 to 500 microns which is administered in the manner in which snuff is taken, i.e. by rapid inhalation through the

nasal passage from a container of the powder held close up to the nose.

It should be understood that in addition to the aforementioned ingredients the formulations of this invention may include one or more additional ingredients such as diluents, buffers, flavouring agents, binders, surface active agents, thickeners, lubricants, preservatives (including anti-oxidants) and the like.

Where the formulation, for human or for veterinary use is presented in unit dosage form, for example those unit dosage forms specifically mentioned above, each unit thereof conveniently contains the active ingredient (as above defined) in an amount in the range 50 mg to 2 g., and preferably 50 to 200 mg (all weights calculated with reference to the peptide base).

The compounds of this invention preferentially bind to and occupy morphine opiate receptor sites in comparison with their binding to the enkephalin receptor sites. The preferred compounds of this invention are at least 100 times more preferentially bound to morphine receptor sites than to the enkephalin receptor sites.

The compounds of this invention may also be radio-labelled, e.g. with $^{125}I$, $^{3}H$ etc. in a conventional manner and used in binding affinity assays to determine binding of morphine, enkephalins or other peptide compounds to morphine receptor sites. Thus the compounds of this invention of formula I are also useful as intermediates to prepare radioactive labelled compounds which are useful to scientists engaged in mammalian medical studies.

The following Examples serve to illustrate the present invention but should not be construed as in any way providing a limitation thereof. All temperatures are in degree Celsius.

## EXAMPLE 1

## L-Tyrosyl-L-Prolyl-L-Phenylalanyl-L-Thiazolidine-4-carboxyl·amide Acetate (Tyr-Pro-Phe-Thz-$NH_2$·HOAc)

Tyr-Pro-Phe-Thz-$NH_2$·HOAc was synthesized by the solid-phase procedure on a Schwartz/Mann automatic synthesizer. Using a benzhydrylamine resin (BHA, capacity 0.5 meq/g of resin, total 1.5mM/3g of resin) as the solid support, Boc-Thz (1.2g, 5mM) was attached to the resin using dicyclohexylcarbodiimide (DCC) as the coupling reagent. The success of the coupling reactions was monitored by the semi-quantitative ninhydrin test. The following steps were used to couple Boc-Phe, Boc-Pro and Boc-(0-Br-Z)-Tyr to Boc-Thz-BHA resin:

The following abbreviations are used herein:

| | |
|---|---|
| TEA: | Triethylamine |
| TFA: | Trifluoroacetic acid |
| Z: | Carbobenzoxy |
| HOAc: | Acetic Acid |
| n-BUOH: | n-Butanol |
| BHA: | Benzhydrylamine resin, Protein Research Foundation, Osaka, Japan |
| DMF: | Dimethylformamide |
| Bzl: | Benzyl |
| OSu: | Succinimido-oxy |
| BOC: | Benzyloxycarbonyl |

1) washing with $CH_2Cl_2$, 3 x 30 ml, 1 minute
2) prewashing with 40% TFA in $CH_2Cl_2$, 1 x 30 ml, 1 minute
3) deprotection with 40% TFA in $CH_2Cl_2$, 1 x 30 ml, 20 minutes
4) washing with $CH_2Cl_2$, 2 x 30 ml, 1 minute
5) washing with EtOH, 1 x 30 ml, 1 minute
6) washing with $CH_2Cl_2$, 2 x 30 ml, 1 minute
7) prewashing with 10% TEA in $CH_2Cl_2$, 1 x 30 ml, 1 minute

8) neutralization with 10% TEA in $CH_2Cl_2$, 1 x 30 ml, 10 minutes

9) washing with $CH_2Cl_2$, 3 x 30 ml, 1 minute

10) protected amino acid (5mM) in DMF (10 ml) and $CH_2Cl_2$ (20 ml) was added

11) DCC in $CH_2Cl_2$ (0.5M, 10 ml) was added and the reaction time was up to two hours

12) washing with $CH_2Cl_2$, 3 x 30 ml, 1 minute

The resulting Boc-(O-Br-Z)-Tyr-Pro-Phe-Thz-BHA resin was washed well with 40% TFA in $CH_2Cl_2$, $CH_2Cl_2$ and then absolute EtOH respectively. After drying _in vacuo_ overnight, this protected tetrapeptide-BHA resin was cleaved by HF (50 ml) in the presence of anisole (10 ml) for one hour at 0°C. The reaction mixture was evaporated _in vacuo_ to dryness and washed well with anhydrous ether. The desired tetrapeptide was dissolved in 1M HOAc and the resin was filtered off. The resin was rinsed well with 1M HOAc and the combined filtrate was lyophilized to give crude Tyr-Pro-Phe-Thz-$NH_2$ (500 mg). This crude tetrapeptide was purified by counter-current distribution using n-BuOH:HOAc:$H_2O$ (4:1:5 v/v) as the partition solvent (200 transfers) to afford pure Tyr-Pro-Phe-Thz-$NH_2$·HOAc (256 mg, yield 32% based on the total capacity of the BHA resin).

Thin layer chromatography: 50 μg of sample, spray with ninhydrin.

$R_f^1$ (silica gel plate, 5 x 20 cm, n-BuOH:EtOAc:HOAc:$H_2O$ 1:1:1:1) 0.58

$R_f^2$ (silica gel plate, 5 x 20 cm, n-BuOH:pyridine:HOAc:$H_2O$ 15:10:3:12) 0.56

Electrophoresis: 100 μg of sample, Whatman 3MM paper, pH 5.6 pyridine-acetate buffer, 1000V, 80 minutes. Single spot migrating toward the cathode. $R_f$ = 0.75 with reference to picric acid.

Amino acid analysis: 6N HCl at 110°C *in vacuo* for 20 hours Tyr 0.93, Pro 1.05, Phe 1.02 and Thz 0.82. 86% of peptide

EXAMPLE 2

L-Tyrosyl-L-Prolyl-N-Methyl-L-Phenylalanyl-D-Prolinamide· Acetate (Tyr-Pro-N-Me-Phe-D-Pro-$NH_2$·HOAc)

In the solid phase synthesis of Tyr-Pro-N-Me-Phe-D-Pro-$NH_2$, Boc-D-Pro (10mM) was attached to a benzhydrylamine resin (6 g 0.5 meq/g of resin) using dicyclohexylcarbodiimide (DCC) as the coupling reagent. To this Boc-D-Pro-BHA resin, the following protected amino acids were successively added, Boc-N-Me-Phe, Boc-Pro and Boc-(O-Br-Z)-Tyr. A 3.0 molar excess of each protected amino acid was used. The condition for introducing the protected amino acids were the same as those described above. The final protected tetrapeptide-BHA resin was dried *in vacuo* and cleaved by HF (100ml) with distilled anisole (10ml) at 0°C for one hour to give the crude tetrapeptide. This peptide was also purified by counter-current distribution using n-BuOH:HOAc:$H_2O$ (4:1:5 V/V) with 200 transfers to afford pure Tyr-Pro-N-Me-Phe-D-Pro-$NH_2$·HOAc (691 mg yield 43% based on the total capacity of BHA resin).

Thin-layer chromatography: 50μg of sample, spray with ninhydrin

$R_f^1$ (cellulose plate, 5 x 20 cm, n-BuOH:pyridine:HOAc:$H_2O$ 15:10:3:12) 0.87

$R_f^2$ (cellulose plate, 5 x 20 cm, n-BuOH:pyridine:HOAc:$H_2O$ 42:24:4:30) 0.81

$R_f^3$ (cellulose plate, 5 x 20 cm, n-BuOH:HOAc:$H_2O$ 4:1:5 upper layer) 0.70

Electrophoresis: 100 μg of sample, Whatman 3MM paper, pH 5.6 pyridine-acetate buffer, 1000V 90 minutes. Single Pauly positive spot migrating toward the cathode.

$R_f$ 0.65 with reference to picric acid.

Amino acid analysis: 6N HCl at 110°C for 20 hours in vacuo. Tyr 0.96, Pro 2.00 and $NH_3$ 0.92. 85% of peptide.

EXAMPLE 3

L-Tyrosyl-L-Prolyl-N-Methyl-L-Phenylalanyl-D-Prolinol·Acetate (Tyr-Pro-N-Me-Phe-D-Pro-ol·HOAc)

The solution synthesis of Tyr-Pro-N-Me-Phe-D-Pro-ol was performed as described by the following:

D-Pro-ol·Trifluoroacetate

Boc-D-Pro (3 g) in dry tetrahydrofuran (100 ml) containing lithium aluminium hydride (1 g) was stirred at room temperature during 15 hours. The tetrahydrofuran was then evaporated under reduced pressure and the excess lithium aluminium hydride was decomposed using saturated aqueous sodium potassium tartrate. This was then extracted with ethyl acetate which was rinsed with water. The organic layer was dried with magnesium sulphate, was filtered and was evaporated to the crude Boc-D-Pro-ol which was purified by chromatography on silica gel and elution with benzene-chloroform (50% v/v) afforded oily Boc-D-Pro-ol (1.2 g, yield 46%). This Boc-D-Pro-ol was treated with trifluoroacetic acid in chloroform (40% v/v) at room temperature for 20 minutes. After evaporation in vacuo to dryness, TFA·D-Pro-ol was obtained in quantitative yield (1.1 g). The $R_f$ of this compound was identical with the authentic sample of Pro-ol (silica gel plate, $CHCl_3$:MeOH 8:2 v/v).

Boc-N-Me-Phe-D-Pro-ol.

Boc-N-Me-Phe (1.25 g, 4.5 mM) and 1-hydroxybenzotriazole (0.75 g), in dry DMF (20 ml), was treated with DCC (1.3 g) at 0°C. After being stirred at room temperature for 2 hours, TFA·D-Pro-ol (1.1 g) and triethylamine (3 ml) in DMF (20 ml) was added. After

another 18 hours at room temperature, the reaction mixture was filtered and the filtrate was evaporated in vacuo to dryness. The residue was dissolved in ethyl acetate and washed well with dilute HCl and water respectively. The organic layer was dried with anhydrous sodium sulphate and filtered, and evaporated to give crude protected dipeptide (1.9 g). This was purified by a chromatography on silica gel and eluted with chloroform to afford pure Boc-N-Me-Phe-D-Pro-ol (500 mg, yield 30%).

TFA.N-Me-Phe-D-Pro-ol.

Boc-N-Me-Phe-D-Pro-ol (500 mg) was treated with trifluoroacetic acid in chloroform (40% v/v) at room temperature for 20 minutes, followed by evaporation in vacuo to dryness. TFA.N-Me-Phe-D-Pro-ol yielded quantitatively.

Boc-Tyr(2,6-Di-Cl-Bzl)-Pro.

Boc-Tyr(2,6-Di-Cl-Bzl)-OSu (5 g, 8.7mM) and Pro (2.2 g, 19 mM), in triethylamine (3.5 ml) and DMF (100 ml) were stirred at room temperature for 24 hours. The reaction mixture was evaporated to dryness in vacuo and the residue was dissolved in ethyl acetate. The organic layer was rinsed with 1N HCl and water respectively. This was dried with anhydrous $MgSO_4$, filtered and evaporated to afford the crude protected dipeptide which was purified by chromatography on silica gel with elution by $CHCl_3$ to give pure Boc-Tyr(2,6-Di-Cl-Bzl)-Pro (2.2 g, yield 47%).

Boc-Tyr(2,6-Di-Cl-Bzl)-Pro-N-Me-Phe-D-Pro-ol.

Boc-Tyr(2,6-Di-Cl-Bzl)-Pro (750 mg, 1.4 mM) and TFA.N-Me-Phe-D-Pro-ol (500 mg, 1.0 mM), in dry DMF (50 ml) and triethylamine (0.3 ml), were treated with DCC (0.5M in $CH_2Cl_2$, 4 ml) at 0°C. After being stirred at room temperature overnight, the reaction mixture was evaporated in vacuo. The residue was dissolved in ethyl acetate which was washed well with 2.5% HCl, water, 5%

sodium carbonate and water respectively. The organic layer was dried with anhydrous sodium sulphate, filtered and evaporated to dryness. Protected tetrapeptide (1.1 g) was collected and purified by silica gel chromatography eluting $CHCl_3$ to afford pure Boc-Tyr(2,6-Di-Cl-Bzl)-Pro-N-Me-Phe-D-Pro-ol (560 mg, yield 62%).

Tyr-Pro-N-Me-Phe-D-Pro-ol.HOAc.

Boc-Tyr(2,6-Di-Cl-Bzl)-Pro-N-Me-Phe-D-Pro-ol (560 mg. 0.62 mM) was cleaved by HF (10 ml) in the presence of distilled anisole (1 ml) at 0°C for one hour. After being evaporated in vacuo to dryness, the crude tetrapeptide was purified by counter-current distribution using n-BuOH: HOAc:$H_2O$ (4:1:5 v/v) as the partition solvents with 200 transfers to give pure Tyr-Pro-N-Me-Phe-D-Pro-ol.HOAc (130 mg, yield 40%).

Thin-layer chromatography: 50 g of sample, ninhydrin spray.

$R_f^1$ (silica gel plate, 5 x 20 cm, n-BuOH:HOAc:EtOAc:$H_2O$ 1:1:1:1) 0.54.

$R_f^2$ (silica gel plate, 5 x 20 cm, n-BuOH:pyridine:HOAc:$H_2O$ 15:10:3:12) 0.64.

Electrophoresis: 100 g of sample, Whatman 3MM paper, pH 5.6 pyridine-acetate buffer, 1000V, one hour. Single spot migrating toward the cathode.

$R_f$ = 0.64 with reference to picric acid.

Amino acid analysis: 6N HCl at 110°C. for 20 hours in vacuo.

Tyr 0.86, Pro 0.90, N-Me-Phe 1.05 and Pro-ol 1.19.

76% of peptide.

Using the method of Example 1, the following acetate salt of peptides were prepared.

## EXAMPLE 4

L-Tyrosyl-L-Prolyl-L-Phenylalanyl-D-Leucinamide·Acetate (Tyr-Pro-Phe-D-Leu-$NH_2$·HOAc)

Thin-layer chromatography: 50μg of sample. Spray with ninhydrin

$R_f^1$ (silica gel plate, 5 x 20 cm, n-BuOH:EtOAc:HOAc:$H_2O$ 1:1:1:1) 0.65.

$R_f^2$ (silica gel plate, 5 x 20 cm, n-BuOH:pyridine:HOAc:$H_2O$ 15:10 :3:12) 0.55.

Electrophoresis: 100 μg of sample, Whatman 3MM paper, pH 5.6 pyridine-acetate buffer, 1000V, 80 minutes. Single ninhydrin positive spot migrating toward the cathode. $R_f$ = 0.84 with reference to picric acid.
Amino acid analysis: 6N HCl at 110°C for 20 hours *in vacuo*. Tyr 0.98, Pro 1.04, Phe 0.93 and Leu 1.04. 92% of peptide.

EXAMPLE 5

L-Tyrosyl-L-3,4-dehydroProlyl-N-Methyl-L-Phenylalanyl-D-Prolinamide·Acetate (Tyr-3,4-dehydroPro-N-Me-Phe-D-Pro-$NH_2$· HOAc)

Thin-layer chromatography: 50 μg of sample. Spray with ninhydrin

$R_f^1$ (silica gel plate, 5 x 20 cm, EtOAc:pyridine:HOAc:$H_2O$ 1:1:1:1) 0.55

$R_f^2$ (silica gel plate, 5 x 20 cm, n-BuOH:pyridine:HOAc:$H_2O$ 15:10:3:12) 0.60

Electrophoresis: 100 μg of sample, Whatman 3MM paper, pH 5.6 pyridine-acetate buffer, 1000V, 1 hour. Single ninhydrin spot migrating toward the cathode. $R_f$ = 0.77 with reference to picric acid.
Amino acid analysis: 6N HCl at 110°C for 20 hours *in vacuo*. Tyr 1.03, Pro 0.97, 3,4-dehydroPro 0.74 and $NH_3$ 0.96.

EXAMPLE 6

L-Tyrosyl-L-4-hydroxyProlyl-N-Methyl-Phenylalanyl-D-Prolinamide ·Acetate (Tyr-4-OH-Pro-N-Me-Phe-D-Pro-$NH_2$·HOAc)

Thin-layer chromatography: 50 μg of sample. Spray with ninhydrin.

$R_f^1$ (silica gel plate, 5 x 20 cm, EtOAc:n-BuOH:HOAc:$H_2O$ 1:1:1:1) 0.56

$R_f^2$ (silica gel plate, 5 x 20 cm, n-BuOH:pyridine:HOAc:$H_2O$ 15:10:3:12) 0.60

Electrophoresis: 100 µg of sample, Whatman 3MM paper, pH 5.6 pyridine-acetate buffer, 1000V, 1 hour. Single ninhydrin positive spot migrating toward the cathode. $R_f$ = 0.71 with reference to picric acid.

Amino Acid analysis: 6N HCl at 110°C for 20 hours *in vacuo*. Tyr 1.03, Pro 0.96, $NH_3$ 1.01.

EXAMPLE 7

L-Tyrosyl-L-Prolyl-N-Methyl-L-Phenylalanyl-D-Proline·Acetate (Tyr-Pro-N-Me-Phe-D-Pro·HOAc)

Thin-layer chromatography: 50 µg of sample. Spray with ninhydrin.

$R_f^1$ (silica gel plate, 5 x 20 cm, EtOAc:n-BuOH:HOAc:$H_2O$ 1:1:1:1) 0.59

$R_f^2$ (silica gel plate, 5 x 20 cm, n-BuOH:pyridine:HOAc:$H_2O$ 15:10:3:12) 0.57

$R_f^3$ (silica gel plate, 5 x 20 cm, n-BuOH:HOAc:$H_2O$ 4:1:5 upper layer) 0.39

Electrophoresis: 100 µg of sample, Whatman 3MM paper, pH 5.6 pyridine-acetate buffer, 1000V, 1 hour. Single ninhydrin positive spot at neutral area. $R_f$ = 0.20 with reference to picric acid.

Amino acid analysis: 6N HCl at 110°C for 20 hours *in vacuo*. Tyr 0.97, Pro 2.03, 68% of peptide.

EXAMPLE 8

N-Methyl-L-Tyrosyl-L-Prolyl-N-Methyl-L-Phenylalanyl-D-Prolinamide Acetate (N-Me-Tyr-Pro-N-Me-Phe-D-Pro-$NH_2$·HOAc)

Thin-layer chromatography: 50 µg of sample. Spray with

o-tolidine.

$R_f^1$ (cellulose plate, 5 x 20 cm, n-BuOH:pyridine:HOAc:$H_2O$ 15:10:3:12) 0.91

$R_f^2$ (cellulose plate, 5 x 20 cm, n-BuOH:HOAc:$H_2O$ 4:1:5 upper layer) 0.75

$R_f^3$ (cellulose plate, 5 x 20 cm, n-BuOH:pyridine:HOAc:$H_2O$ 42:24:4:30) 0.79

Electrophoresis: 100 µg of sample, Whatman 3MM paper, pH 5.6 pyridine-acetate buffer, 1000V, 90 minutes. Single o-tolidine positive spot migrating toward the cathode. $R_f$ = 0.88 with reference to picric acid.

Amino acid analysis: 6N HCl at 110°C for 20 hours *in vacuo*. Pro 2.00. 79% of peptide.

EXAMPLE 9

L-Tyrosyl-L-Prolyl-N-Methyl-L-Phenylalanyl-L-Proliamide· Acetate (Tyr-Pro-N-Me-Phe-Pro-$NH_2$·HOAc)

Thin-layer chromatography: 50 µg of sample. Spray with Pauly reagent.

$R_f^1$ (cellulose plate, 5 x 20 cm, n-BuOH:pyridine:HOAc:$H_2O$ 15:10:3:12) 0.83

$R_f^2$ (cellulose plate, 5 x 20 cm, n-BuOH:pyridine:HOAc:$H_2O$ 42:24:4:30) 0.78

Electrophoresis: 100 µg of sample, Whatman 3MM paper, pH 5.6 pyridine-acetate buffer, 1000V, 90 minutes. Single Pauly positive spot migrating toward the cathode. $R_f$ = 0.70 with reference to picric acid.

Amino acid analysis: 6N HCl at 110°C for 20 hours *in vacuo*. Tyr 0.74, Pro 2.26. 68% of peptide.

EXAMPLE 10

L-Tyrosyl-L-Prolyl-N-Methyl-Phenylalanyl-D-Prolyl-Glycinamide Acetate (Tyr-Pro-N-Me-Phe-D-Pro-Gly-$NH_2$·HOAc)

Thin-layer chromatography: 50 µg of sample. Spray with Pauly reagent.

$R_f^1$ (cellulose plate, 5 x 20 cm, n-BuOH:pyridine:HOAc:$H_2O$ 15:10:3:12) 0.81

$R_f^2$ (cellulose plate, 5 x 20 cm, n-BuOH:pyridine:HOAc:$H_2O$ 42:24:4:30) 0.78

Electrophoresis: 100 µg of sample, Whatman 3MM paper, pH 5.6 pyridine-acetate buffer, 1000V, one hour. Single spot migrating toward the cathode.

$R_f$ = 0.54 with reference to picric acid.

Amino acid analysis: 6N HCl at 110°C for 20 hours *in vacuo*. Tyr 0.95, Pro 2.07, Gly 0.98 and $NH_3$ 0.91. 73% of peptide.

## EXAMPLE 11 (Analgesic Activity)

The acetate salts of formula I described in Examples 1 to 3 were tested for analgesic activity in the rat tail withdrawal assay. In this test, latencies to withdraw the tail from hot (55°C) water are measured 5 minutes, 30 minutes, 60 minutes and 90 minutes after administration of the acetate salts. Latencies of 5 seconds or greater indicate strong analgesic activity and 20 seconds is a maximum response in this test. The peptides were administered intracisternally or intravenously.

### A. Intracisternal Administration

The acetate salt was dissolved in 10 µl of sterile saline and injected intracisternally into the rat brain.

(1) The formula I acetates of Examples 1 and 3 were administered at a dose of 10 µg per rat brain. The results are tabulated:

| Compound of Example | Time after Injection (min) | Latency of Tail Withdrawal (sec) |
|---|---|---|
| 1 | 15 | 4.5 ± 0.5 |
| | 30 | 4.6 ± 0.8 |
| | 60 | 4.6 ± 0.8 |
| 3 | 15 | 10.6 ± 4.0 |
| | 30 | 11.2 ± 4.6 |
| | 60 | 9.0 ± 3.0 |

(2) The compound of Example 2 was administered at several doses. The results are tabulated:

| Dose | N | Latency of Tail Withdrawal | Duration (min.) | Observation |
|---|---|---|---|---|
| 20μg | 2 | 20 | >90 | Symptoms |
| 10μg | 2 | 20 | >90 | Symptoms |
| 10μg + Naloxone (4 mg/kg i.p) | 1 | <5 | - | No Symptoms |
| 1μg | 2 | 20 | >90 | Cataleptic |
| 0.1μg | 2 | 5-7 | 30<br>60 | No Symptoms |

N = No. of animals

Symptoms = slow respiration, exophthalmus, "lead pipe" rigidity.

Cataleptic = catalepsy plus exophthalmus

B. Intravenous Administration

The formula I acetate of Example 2 was dissolved in a sufficient sterile, distilled water to give an injection volume of 1 ml/kg of body weight and administered to rats as a bolus i.v. injection at several dosages. Results are tabulated:

| Dose (mg/kg) | N | Time after Injection (min.) | Latency of Tail Withdrawal (sec.) | Comments |
|---|---|---|---|---|
| 2.5 | 4 | 5 | 2 | |
| | | 15 | 3.5 | |
| | | 30 | 3.0 | |
| | | 60 | 2.5 | |
| 5.0 | 4 | 5 | 8.4 ± 3.6 | Individual |
| | | 15 | 10.3 ± 3.1 | rats showed |
| | | 30 | 9.1 ± 3.4 | 20 sec. |
| | | 60 | 7.8 ± 3.0 | latencies and |
| | | | | morphine-like |
| | | | | symptoms |
| 10.0 | 6 | 5 | 10.2 ± 3.2 | Same |
| | | 15 | 11.1 ± 3.1 | as |
| | | 30 | 7.5 ± 2.5 | above |
| | | 60 | 6.4 ± 2.3 | |

A morphine control was also administered intravenously with the following results:

| Dose (mg/kg) | N | Time after Injection (min.) | Latency of Tail Withdrawal (sec.) | Comments |
|---|---|---|---|---|
| 2.5 | 3 | 15 | 5 | |
| | | 30 | 6 | |
| | | 60 | 4.1 | |
| 5.0 | 3 | 15 | 20 | Catalepsy |
| | | 30 | 20 | Rigidity |
| | | 60 | 20 | |
| 7.5 | 3 | 15 | 20 | Same |
| | | 30 | 20 | As |
| | | 60 | 20 | Above |

EXAMPLE 12 (Pharmaceutical Formulations)

A. Tablet (20 mg/tablet)

| | |
|---|---|
| Formula I acetate of Example 2 | 20 mg |
| Lactose | 76 mg |
| Maize starch | 10 mg |
| Polyvinylpyrrolidone | 2 mg |
| Magnesium Stearate | 2 mg |

The compound of Example 2, lactose and maize starch are mixed together and granulated with a solution of the polyvinylpyrrolidone dissolved in 50% aqueous ethanol. The granules are dried, the magnesium stearate added, and the mixture compressed to produce tablets, 110 mg per tablet.

B. Capsule (20 mg/capsule)

| | |
|---|---|
| Formula I acetate of Example 1 | 20 mg |
| Lactose | 190 mg |
| Maize starch | 189 mg |
| Magnesium stearate | 1 mg |

The ingredients are mixed and filled into No. 1 hard gelatin capsules giving capsules having a fill weight of 400 mg per capsule.

C. Suppository (5 mg/product)

| | | |
|---|---|---|
| Formula I acetate of Example 2 | | 250 mg |
| Suppository base (Massa Esterinum C) | q.s. to | 100 g |

The suppository base is melted at 40°C. The compound of Example 2 is gradually incorporated as fine powder form and mixed until homogeneous. The mixture is then poured into moulds, 2 g per mould, and allowed to set.

Massa Esterinum C is a commercially available suppository base consisting of a mixture of mono- di- and triglycerides of saturated fatty acids. It is marketed by Henkel International, Dusseldorf, West Germany.

D. Pessary (5 mg/product)

| | |
|---|---|
| Formula I acetate of Example 3 | 5 mg |
| Lactose | 400 mg |
| Polyvinylpyrrolidone | 5 mg |
| Magnesium stearate | 5 mg |

The compound of Example 3 and the lactose are mixed together and granulated with a solution of the polyvinylpyrrolidone in 50% aqueous ethanol. The granules are dried and, after addition of the magnesium stearate, compressed on suitably shaped punches, 415 mg per pessary.

E. Freeze-dried Injection (100 mg/vial)

| | |
|---|---|
| Formula I acetate of Example 2 | 100 mg |
| Water for injection q.s. to | 2.0 ml |

The compound of Example 2 is dissolved in the water for injection. The solution is sterilised by passage through a membrane filter, 0.2µm pore size. The sterile filtrate is filled into sterile glass vials, 2 ml/vial, under aseptic conditions and freeze-dried. The vials are closed with sterile rubber closures secured with an aluminium seal.

The injection is reconstituted prior to administration by the addition of a convenient volume of water for injection or sterile saline solution:

CLAIMS

1. A peptide of the formula (I):

$$A - B - C - Z - (R^2)_n \qquad (I)$$

where n is O or 1;

A is Tyr, N-alkyl-Tyr where the alkyl group has 1 to 4 carbon atoms, and is preferably methyl or ethyl or N-alkenyl-Tyr where the alkenyl group has 2 to 4 carbon atoms, and is preferably allyl, the Tyr in each case having L chirality;

B is Pro(L), 3,4-dehydro-Pro(L), 4-hydroxy-Pro(L), Thz (L) or Pip(L);

C is Phe(L or D), N-alkyl-Phe (L or D), α-alkyl-Phe(L or D), in each of the above the alkyl group having 1 to 4 carbons and is preferably methyl.

Z is Pro(L or D), Leu(L or D), Thz(L or D), Tyr (L or D), Pip (L or D), Hse (L or D), Nva (L or D); 4-hydroxy-Pro (L or D), Met (L or D), $Met(O_2)$ (L or D), Ile (L or D), Nle(L or D); Met(O) (L or D), Gly, Abu (L or D), Val (L or D), Aib, 3,4-dehydro-Pro (L or D), cyclo-Leu (L or D);

$R^2$ is Gly-$NH_2$, $-OR^5$, (where $R^5$ is an alkyl group of 1 to 4 carbon atoms, preferably methyl or ethyl), or a group $-NR^6R^7$ (where $R^6$ and $R^7$ are the same or different and each is hydrogen or an alkyl group of 1 to 4 carbon atoms preferably methyl) and, when n is 0, the alcohols resulting from reduction of the peptide A - B - C - Z ; and pharmaceutically acceptable salts thereof ; except that where n is 1 and A is Tyr and B is Pro and C is Phe, and Z is Pro, $R^2$ is not $NH_2$ and except that where n is 0, A is Tyr and B is Pro, and C is Phe, Z is not Pro ;

in the above L and D defining the chirality of the amino acid.

2. A peptide or salt according to claim 1 wherein A is Tyr, B is Pro, C is Phe or N-Me-Phe and Z is Pro, Pro.$NH_2$, Pro-ol, Leu-$NH_2$ or Thz-$NH_2$.

3. A peptide or salt according to claim 1 or 2 wherein n is 0 or wherein n is 1 and $R^2$ is Gly$NH_2$.

4. Tyr-Tyr-Pro-N-Me-Phe-D-Pro-$NH_2$ and pharmaceutically acceptable salts thereof.

5. Tyr-Pro-N-Me-Phe-D-Pro-ol and pharmaceutically acceptable salts thereof.

6. Tyr-Pro-Phe-Thz-$NH_2$ and pharmaceutically acceptable salts thereof.

7. Tyr-Pro-Phe-D-Leu-$NH_2$,
Tyr-3, 4-dehydro-Pro-N-Me-Phe-D-Pro-$NH_2$,
Tyr-4-hydroxy-Pro-N-Me-Phe-D-Pro-$NH_2$,
Tyr-Thz-N-Me-Phe-D-Pro-$NH_2$,
Tyr-Pip-N-Me-Phe-D-Pro-$NH_2$,
Tyr-Pro-N-Me-Phe-D-Pro,
N-Me-Tyr-Pro-N-Me-Phe-D-Pro-$NH_2$,
Tyr-Pro-N-Me-Phe-Pro-$NH_2$,
Tyr-Pro-N-Me-Phe-D-Pro-Gly-$NH_2$
and pharmaceutically acceptable salts thereof.

8. An acetate of a peptide according to any one of the preceding claims

9. A peptide or salt according to any one of the preceding claims wherein the chirality of residue C is L.

10. A process for the preparation of a peptide or salt as defined in any one of the preceding claims which comprises condensing E-OH with H - Y under conditions to form a

-CONH bond, where E is A-, A-B- or A-B-C— or, when n is 1 and $R^2$ is Gly-$NH_2$, E may be A-B-C-Z— and Y is -B-C-Z-$(R^2)_n$, -C-Z-$(R^2)_n$; -Z-$(R^2)_n$ or Gly$NH_2$ respectively, the terminal amino group of E-OH and the terminal carboxyl group of Z being optionally protected and the carboxylic group of E-OH being optionally activated, followed by the steps of removing any protecting groups present and optionally, converting a free base and/or free acid product into a salt or ester or amide or converting a salt product into another salt product or a free acid and/or free base product.

11. A composition comprising a peptide or salt according to any one of claims 1 - 9 together with a pharmaceutically or veterinarily acceptable carrier.

12. A composition according to claim 11 in unit dose form.

13 A compound or salt according to any one of claims 1-9 or a composition according to claim 11 or 12 for use in a method of treatment of the human or animal body by surgery or therapy or for use in diagnostic methods practiced on the human or animal body.

European Patent Office

# EUROPEAN SEARCH REPORT

0053029
Application number

EP 81305519.1

| Category | Citation of document with indication, where appropriate, of relevant passages | Relevant to claim | CLASSIFICATION OF THE APPLICATION (Int. Cl.3) |
|---|---|---|---|
| | DOCUMENTS CONSIDERED TO BE RELEVANT | | C 07 C 103/52<br>A 61 K 37/02 |
| P | HOPPE SEYLER'S Z. PHYSIOL. CHEM. vol. 361 1980, Berlin, New York,<br>F. LOTTSPEICH et al. "Novel opioid peptides derived from casein (β-casomorphins) III. synthetic peptides corresponding to components from bovine casein peptone", pages 1835-1839<br>* Pages 1835-1838 *<br>-- | 1-13 | |
| P | EP - A1 - 0 025 218 (TROPONWERKE GMBH & CO.KG)(18-03-1981)<br>* Pages 1-3,7 *<br>& WO-A1-81/00712 (19-03-1981)<br>& DE-A1-2 936 099 (02-04-1981)<br>-- | 1-13 | TECHNICAL FIELDS SEARCHED (Int. Cl.3)<br>C 07 C 103/00 |
| | HOPPE SEYLER'S Z. PHYSIOL. CHEM. vol. 360 1979, Berlin, New York,<br>A. HENSCHEN et al. "Novel opioid peptides derived from casein (β-casomorphins) II. structure of active components from bovine casein peptone" pages 1217-1224<br>* Pages 1217,1219,1222,1223 *<br>-- | 1-7,9-13 | |
| | CHEMICAL ABSTRACTS vol. 93, 1980<br>E.T. WEI et al. "Cardiovascular effects of peptides related to the enkephalins and β-casomorphin" 61992s<br>* Lines 17-23 *<br>-- | 1-5,9-13 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | US - A - 4 028 319 (JONES)<br>* Examples 28-35 *<br>---- | 1,2,7,9-13 | |
| X | The present search report has been drawn up for all claims | | &: member of the same patent family,<br>corresponding document |

| Place of search | Date of completion of the search | Examiner |
|---|---|---|
| VIENNA | 08-02-1982 | PETROUSEK |